# EUROPEAN PATENT SPECIFICATION

(11) **EP 4 380 637 B1**
(45) Date of publication and mention of the grant of the patent: **27.08.2025**
(21) Application number: 22761417.9
(22) Date of filing: 28.07.2022
(51) Int. Cl.: A61L 9/12

(54) **DEVICE FOR EVAPORATING VOLATILE SUBSTANCES**
VORRICHTUNG ZUM VERDAMPFEN VON FLÜCHTIGEN STOFFEN
DISPOSITIF D'ÉVAPORATION DE SUBSTANCES VOLATILES

(30) Priority: 05.08.2021 ES 202130763
(43) Date of publication of application: 12.06.2024
(73) Proprietor: Zobele Holding SpA, 38100 Trento (IT)
(72) Inventor: PROKOP, Matthew, 08019 BARCELONA (ES); SANCHO MARZO, Alberto José, 08019 BARCELONA (ES); RAMÍREZ ALVARADO, Jesús Orlando, 08019 BARCELONA (ES)
(74) Representative: Herrero & Asociados, S.L.
(86) International application number: PCT/EP2022/071285
(87) International publication number: WO 2023/012041

(56) References cited:
- WO-A1-2008/005182
- WO-A2-2004/105815
- US-A1- 2002 068 009
- US-A1- 2002 172 512

## Description

### Field of the invention

The present invention relates to a device for evaporating volatile substances, which allows the evaporation rate to be regulated.

### Background to the invention

US2002172512 discloses a heated wick-type air freshener according to the preamble of claim 1.

Currently known evaporation devices for volatile substances usually comprise means for regulating the evaporation rate of the volatile substances, depending on the user's preferences.

These devices for evaporation of volatile substances usually comprise an electric heater, which heats a wick impregnated with the volatile substances.

For example, one such means of regulating the evaporation rate consists of a rotating part with a window which, in a position of maximum evaporation, exposes the wick directly to the heater and, in a position of minimum evaporation, blocks direct exposure between the wick and the heater.

It has been shown in tests that these means of regulation are ineffective in changing the evaporation rate and thus the perceived intensity of fragrance.

Other means of evaporation rate regulation involving movement of the heater with respect to the wick, either vertically or radially, are also known.

These prior art regulation means involving movement of the heater with respect to the wick is more effective in changing the evaporation rate, but have disadvantages, such as potential damage to the active electrical components due to frequent movement. In addition, the longer length of cables needed to allow such movement is also detrimental to cost, and has a negative impact on the environment, as it requires more material than necessary and adds complexity during assembly.

### Description of the invention

Therefore, an objective of the present invention is to provide a device for evaporating volatile substances, which allows changing the area of the air flow directly around the wick through the linear movement of a rotating part, thus affecting the evaporation rate and the intensity of the perceived fragrance.

With the device for evaporating volatile substances of the invention, the aforementioned disadvantages are solved, presenting other advantages that will be described below.

The device for evaporating volatile substances according to the present invention comprises:
- a casing;
- a container containing volatile substances;
- an elongated wick defining a longitudinal axis which is partially inserted into the container and which is impregnated with the volatile substances;
- a heater, which heats the wick for evaporating the volatile substances; and
- an evaporation rate regulator, provided with an outlet channel for the volatile substances, the regulator being movable between a first and second positions to regulate the area of the outlet channel;
wherein the regulator is connected to a rotating disc so that the rotation of the rotating disc causes the movement of the regulator between the first and second positions, such movement being perpendicular to the longitudinal axis of the wick.

Preferably, the regulator comprises a projection that is housed in a complementary slot of the rotating disc.

Furthermore, according to an embodiment, the slot of the rotating disc is curved and extends from substantially the center of the disc to its edge, in particular, the slot of the rotating disc is curved with a non-constant radius.

Advantageously, the outlet channel has an elongated shape in cross-section.

In addition, the rotating disc preferably comprises a central cylindrical wall provided with a window.

Advantageously, the heater is arranged in a fixed position with respect to the wick.

According to a preferred embodiment, the casing comprises a partition provided with a hole, the hole being located above and in front of the heater.

In particular, the casing preferably comprises two partitions, which define a space for the heater.

According to an embodiment, the rotating disc may comprise an actuating lever.

According to this embodiment, the casing can cover the rotating disc and comprises a slot through which the actuating lever exits.

According to the present invention, as indicated above, a device for evaporating volatile substances is provided, which allows changing the area of the air flow directly around the wick through the linear movement of a rotating part, thus affecting the evaporation rate and the intensity of the perceived fragrance.

The linear movement of the rotating part is achieved through the use of a mechanism that converts the user's rotational movement while the heater remains static. The fact that the heater remains static facilitates the assembly of the components and reduces their complexity, which also reduces their cost.

Therefore, the device according to the present invention does not require movement of the heater or active electrical components, thus providing an optimal fragrance intensity regulation solution without compromising competitive price, sustainability, ease of assembly and, above all, safety.

### Brief description of the drawings

For a better understanding of what has been explained above, some drawings are included in which, schematically and only by way of a non-limiting example, a practical case of embodiment is shown.
Figure 1 is a perspective section view of the device for evaporating volatile substances according to the present invention, according to a first embodiment, at a first position of minimum evaporation rate;
Figure 2 is a perspective section view of the device for evaporating volatile substances according to the present invention, according to the first embodiment, at a second position of maximum evaporation rate;
Figure 3 is a perspective view of the evaporating device according to this first embodiment;
Figure 4 is a perspective section view of the device for evaporating volatile substances according to the present invention, according to a second embodiment, at a first position of minimum evaporation rate;
Figure 5 is a perspective section view of the device for evaporating volatile substances according to the present invention, according to the second embodiment, at a second position of maximum evaporation rate;
Figure 6 is a perspective view of the evaporating device according to a third embodiment;
Figure 7 is a perspective section view of the device for evaporating volatile substances according to the present invention, according to the third embodiment, in a first position of minimum evaporation rate; and
Figure 8 is a perspective section view of the device for evaporating volatile substances according to the present invention, according to the third embodiment, at a second position of maximum evaporation rate.

### Description of preferred embodiments

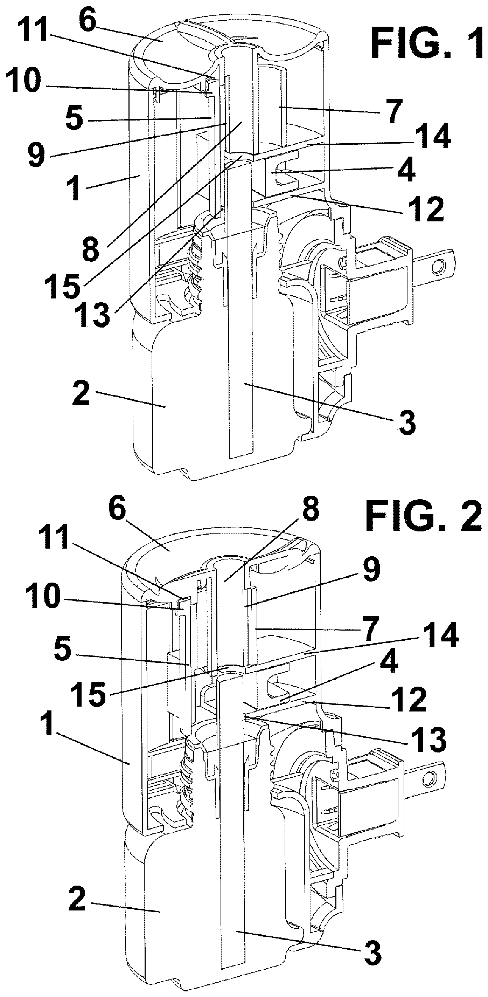

Figures 1 and 2 show a first embodiment of the device for evaporating volatile substances according to the present invention.

This device for evaporating volatile substances comprises a casing 1 inside which the components of the device are suspended in a liquid.

It also comprises a container 2 inside which the volatile substances are housed, and which is removably attached to the casing 1, i.e., it is used as a refill, and it is replaced when the volatile substances inside it have been used up.

The evaporation device also comprises a wick 3, which is elongated and defines a longitudinal axis. The wick 3 is partially housed inside the container 2, so that it is impregnated with the volatile substances for their evaporation.

Next to the end of the wick 3 furthest from the container 2 is a heater 4, which is electrical and connected to the mains via a plug. The function of heater 4 is to heat the end of wick 3 protruding from container 2 in order to evaporate the volatile substances. It should be noted that this heater 4 is fixed to the casing 1, so that it is also fixed with respect to the wick 3.

In the evaporation device according to the present invention the evaporation rate of volatile substances can be regulated.

For this regulation, the device comprises a regulator 5 which is connected to a rotating disc 6.

The regulator 5 comprises an outlet channel 7 and is movable between a first position (position of minimum evaporation rate, shown in figure 1) and a second position (position of maximum evaporation rate, shown in figure 2). It should be noted, however, that the regulator 5 can be placed in any intermediate position between these first and second positions.

This outlet channel 7 is elongated and the displacement of the regulator 5 is substantially perpendicular to the longitudinal axis of the wick 3, as will be explained below.

For its part, the rotating disc 6 comprises a cylindrical wall 8 provided with a window 9. This cylindrical wall 8 extends from the inner central part of the rotating disc 6, remaining inside the outlet channel 7 of the regulator 5, as shown in figures 1 and 2.

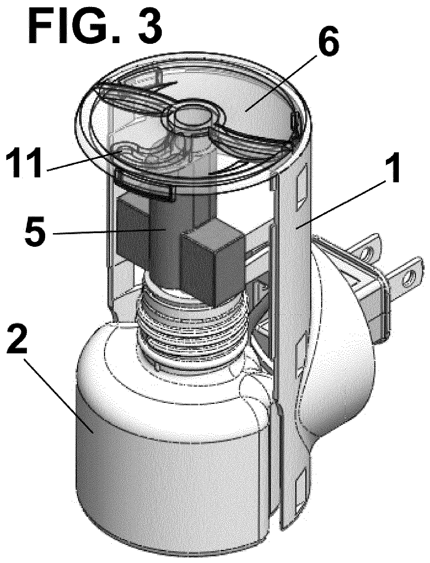

The connection between the regulator 5 and the rotating disc 6 is made by means of a projection 10 of the regulator, which is housed in a slot 11 of the rotating disc 6. This slot 11 is curved, with a non-constant radius, extending from the center of the rotating disc 6 to its edge. This slot 11 can be seen in figure 3.

In this way, when the rotating disc 6 is rotated, the regulator 5 will move from the first position to the second position, or vice versa, or to any intermediate position, describing a straight trajectory substantially perpendicular to the longitudinal axis of the wick 3.

It should also be noted that the casing 1 comprises two partitions 12, 14 provided with respective holes 13, 15 in correspondence with the end of the wick 3, the outlet channel 7 and the cylindrical wall 8, these partitions 12, 14 defining a space where the heater 4 is placed.

For example, in the minimum evaporation rate position, the regulator 5 is positioned approximately 1.5 mm radially from the wick 3. This corresponds to an air flow area of approximately 57 mm² and an evaporation rate of approximately 10 mg/h at steady state.

For example, in the maximum evaporation rate position, the regulator 5 is positioned approximately 6 mm radially from the wick 3. This corresponds to an air flow area of approximately 94 mm² and an evaporation rate of approximately 15 mg/h at steady state.

This evaporation rate ratio of 1:1.5 at minimum to maximum conditions is a noticeable change in intensity for the average consumer.

The operation of the evaporation device according to the present invention is the same as that of conventional evaporation devices.

In particular, the air entering the casing 1 through inlets is mixed with the volatile substances evaporating from the wick 3 and escapes into the environment through the outlet channel 7, the window 9 and the inner part of the cylindrical wall 8.

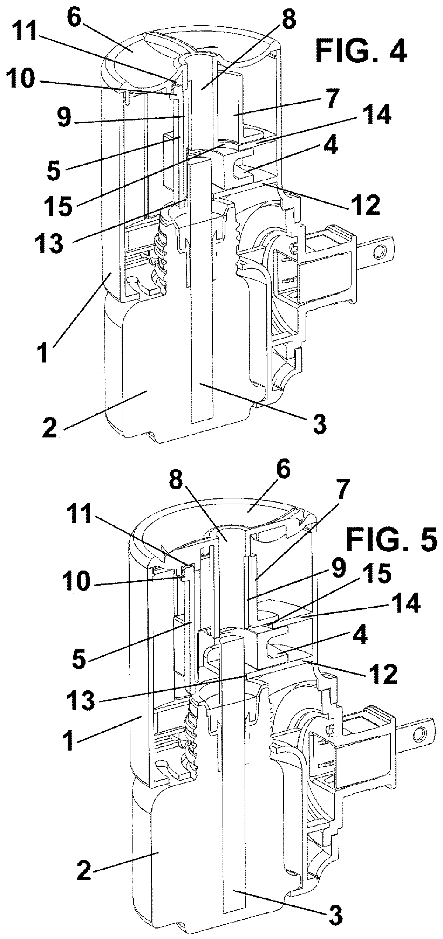

Figures 4 and 5 show a second embodiment of the evaporation device according to the present invention.

For simplicity reasons, the same reference numbers are used to identify the same elements.

Furthermore, the operation of the evaporation device is the same as in the previous embodiment.

The main difference between this second embodiment and the first embodiment is that the hole 15 in the partition 14 is larger and it is positioned directly above and in front of the heater 4.

In this way, the regulator 5 is adapted so that in the position of minimum evaporation rate, shown in figure 4, a part of the hot air is directed out of the wick 3 through the hole 14 and towards the outlet.

In the position of maximum evaporation rate, the regulator 5 covers a part of the hole 13, as shown in figure 5, and the hot air is directed towards the wick 3, thus increasing the evaporation rate.

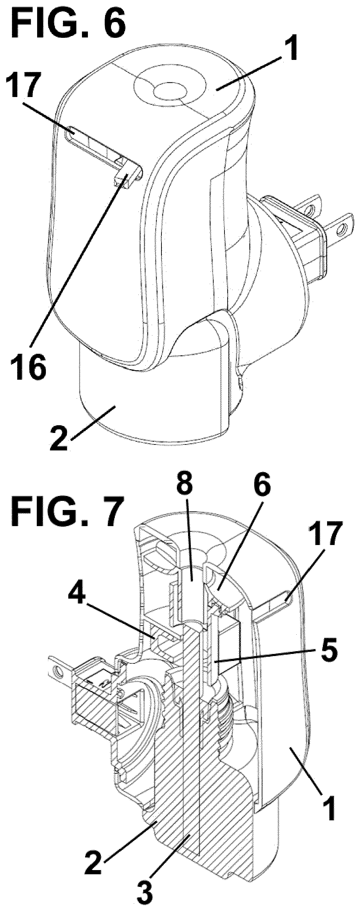

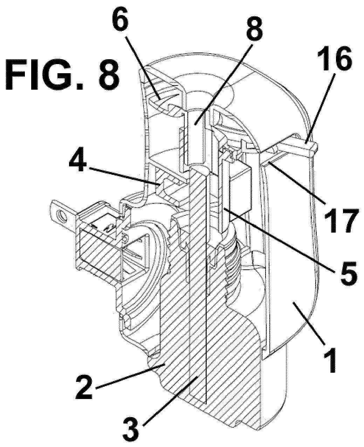

Figures 6 to 8 show a third embodiment of the evaporation device according to the present invention.

For simplicity reasons, the same reference numbers are used to identify the same elements.

Furthermore, the operation of the evaporation device is the same as in the previous embodiments.

The main difference between this third embodiment and the first embodiment is that the rotating disc 6 comprises an actuating lever 16. In addition, the casing 1 covers the rotating disc 6 and comprises a slot 17 through which the actuating lever 16 exits.

In this way, the user positions the actuating lever 16 in a certain position along the slot 17, between a position of maximum evaporation rate and a position of minimum evaporation rate.

Although reference has been made to specific embodiments of the invention, it is obvious to a person skilled in the art that the device for evaporating volatile substances described is susceptible to numerous variations and modifications, and that all the details mentioned can be replaced by technically equivalent ones, without departing from the scope of protection defined by the appended claims.

## Claims

1. A device for evaporating volatile substances, comprising:
- a casing (1);
- a container (2) containing volatile substances;
- an elongated wick (3) defining a longitudinal axis, which is partially inserted into the container (2) and which is impregnated with the volatile substances;
- a heater (4), which heats the wick (3) for evaporation of the volatile substances; and
- an evaporation rate regulator (5), provided with an outlet channel (7) for the volatile substances, the regulator (5) being movable between a first and a second positions for regulating the area of the outlet channel (7);
**characterized in that** the regulator (5) is connected to a rotating disc (6), so that the rotation of the rotating disc (6) causes the regulator (5) to move between the first and second positions, this movement being perpendicular to the longitudinal axis of the wick (3).

2. Device for evaporating volatile substances according to claim 1, wherein the regulator (5) comprises a projection (10) which is housed in a slot (10) complementary to the rotating disc (6).

3. Device for evaporating volatile substances according to claim 2, wherein the slot (10) of the rotating disc (6) is curved and extends from substantially the center of the rotating disc (6) to its edge.

4. Device for evaporating volatile substances according to claim 3, wherein the slot (10) of the rotating disc (6) is curved with a non-constant radius.

5. Device for evaporating volatile substances according to claim 1, wherein the outlet channel (7) has an elongated cross-sectional shape.

6. Device for evaporating volatile substances according to claim 1, wherein the rotating disc (6) comprises a central cylindrical wall (8) provided with a window (9).

7. Device for evaporating volatile substances according to claim 1, wherein the heater (4) is arranged in a fixed position with respect to the wick (3).

8. Device for evaporating volatile substances according to claim 1, wherein the casing (1) comprises a partition (14) provided with a hole (15), the hole (15) being located above and in front of the heater (4).

9. Device for evaporating volatile substances according to claim 8, wherein the casing (1) comprises two partitions (12, 14), which define a space for the heater (4).

10. Device for evaporating volatile substances according to claim 1, wherein the rotating disc (6) comprises an actuating lever (16).

11. Device for evaporating volatile substances according to claim 10, wherein the casing (1) covers the rotating disc (6) and comprises a slot (17) through which the actuating lever (16) exits.

## Patentansprüche

1. Vorrichtung zum Verdampfen flüchtiger Substanzen, umfassend:
- ein Gehäuse (1);
- einen Behälter (2), der flüchtige Substanzen enthält;
- einen länglichen Docht (3), der eine Längsachse definiert, teilweise in den Behälter (2) eingeführt ist und mit den flüchtigen Substanzen imprägniert ist;
- eine Heizvorrichtung (4), die den Docht (3) zur Verdampfung der flüchtigen Substanzen erhitzt; und
- einen Verdampfungsratenregler (5), der mit einem Auslasskanal (7) für die flüchtigen Substanzen versehen ist, wobei der Regler (5) zwischen einer ersten und einer zweiten Position bewegbar ist, um die Fläche des Auslasskanals (7) zu regulieren;
**dadurch gekennzeichnet, dass** der Regler (5) mit einer Rotationsscheibe (6) verbunden ist, so dass die Rotation der Rotationsscheibe (6) bewirkt, dass sich der Regler (5) zwischen der ersten und der zweiten Position bewegt, wobei diese Bewegung senkrecht zu der Längsachse des Dochtes (3) ist.

2. Vorrichtung zum Verdampfen flüchtiger Substanzen nach Anspruch 1, wobei der Regler (5) einen Vorsprung (10) umfasst, der in einem zu der Rotationsscheibe (6) komplementären Schlitz (10) aufgenommen ist.

3. Vorrichtung zum Verdampfen flüchtiger Substanzen nach Anspruch 2, wobei der Schlitz (10) der Rotationsscheibe (6) gekrümmt ist und sich im Wesentlichen vom Zentrum der Rotationsscheibe (6) zu ihrem Rand erstreckt.

4. Vorrichtung zum Verdampfen flüchtiger Substanzen nach Anspruch 3, wobei der Schlitz (10) der Rotationsscheibe (6) mit einem nicht-konstanten Radius gekrümmt ist.

5. Vorrichtung zum Verdampfen flüchtiger Substanzen nach Anspruch 1, wobei der Auslasskanal (7) eine längliche Querschnittsform hat.

6. Vorrichtung zum Verdampfen flüchtiger Substanzen nach Anspruch 1, wobei die Rotationsscheibe (6) eine mit einem Fenster (9) versehene zentrale zylindrische Wand (8) umfasst.

7. Vorrichtung zum Verdampfen flüchtiger Substanzen nach Anspruch 1, wobei die Heizvorrichtung (4) in einer festen Position in Bezug auf den Docht (3) angeordnet ist.

8. Vorrichtung zum Verdampfen flüchtiger Substanzen nach Anspruch 1, wobei das Gehäuse (1) eine mit einem Loch (15) versehene Trennwand (14) umfasst, wobei das Loch (15) oberhalb und vor der Heizvorrichtung (4) angeordnet ist.

9. Vorrichtung zum Verdampfen flüchtiger Substanzen nach Anspruch 8, wobei das Gehäuse (1) zwei Trennwände (12, 14) umfasst, die einen Raum für die Heizvorrichtung (4) definieren.

10. Vorrichtung zum Verdampfen flüchtiger Substanzen nach Anspruch 1, wobei die Rotationsscheibe (6) einen Betätigungshebel (16) umfasst.

11. Vorrichtung zum Verdampfen flüchtiger Substanzen nach Anspruch 10, wobei das Gehäuse (1) die Rotationsscheibe (6) abdeckt und einen Schlitz (17) umfasst, durch den der Betätigungshebel (16) austritt.

## Revendications

1. Dispositif d'évaporation de substances volatiles, comprenant :
- un boîtier (1) ;
- un récipient (2) contenant des substances volatiles ;
- une mèche allongée (3) définissant un axe longitudinal, partiellement insérée dans le récipient (2) et imprégnée des substances volatiles ;
- un élément chauffant (4), qui chauffe la mèche (3) pour provoquer l'évaporation des substances volatiles ; et
- un régulateur de débit d'évaporation (5), muni d'un canal de sortie (7) pour les substances volatiles, le régulateur (5) étant mobile entre une première et une seconde position afin de réguler la section du canal de sortie (7) ;
**caractérisé en ce que** le régulateur (5) est relié à un disque rotatif (6), de sorte que la rotation du disque rotatif (6) entraîne le déplacement du régulateur (5) entre la première et la seconde position, ce déplacement étant perpendiculaire à l'axe longitudinal de la mèche (3).

2. Dispositif d'évaporation de substances volatiles selon la revendication 1, dans lequel le régulateur (5) comprend une saillie (10) logée dans une fente (10) complémentaire au disque rotatif (6).

3. Dispositif d'évaporation de substances volatiles selon la revendication 2, dans lequel la fente (10) du disque rotatif (6) est courbée et s'étend sensiblement du centre du disque rotatif (6) jusqu'à son bord.

4. Dispositif d'évaporation de substances volatiles selon la revendication 3, dans lequel la fente (10) du disque rotatif (6) est courbée selon un rayon non constant.

5. Dispositif d'évaporation de substances volatiles selon la revendication 1, dans lequel le canal de sortie (7) présente une forme de section transversale allongée.

6. Dispositif d'évaporation de substances volatiles selon la revendication 1, dans lequel le disque rotatif (6) comprend une paroi cylindrique centrale (8) munie d'une ouverture (9).

7. Dispositif d'évaporation de substances volatiles selon la revendication 1, dans lequel l'élément chauffant (4) est disposé dans une position fixe par rapport à la mèche (3).

8. Dispositif d'évaporation de substances volatiles selon la revendication 1, dans lequel le boîtier (1) comprend une cloison (14) munie d'un orifice (15), l'orifice (15) étant situé au-dessus et en face de l'élément chauffant (4).

9. Dispositif d'évaporation de substances volatiles selon la revendication 8, dans lequel le boîtier (1) comprend deux cloisons (12, 14) délimitant un espace pour l'élément chauffant (4).

10. Dispositif d'évaporation de substances volatiles selon la revendication 1, dans lequel le disque rotatif (6) comprend un levier d'actionnement (16).

11. Dispositif d'évaporation de substances volatiles selon la revendication 10, dans lequel le boîtier (1) recouvre le disque rotatif (6) et comprend une fente (17) à travers laquelle sort le levier d'actionnement (16).
